# EUROPEAN PATENT SPECIFICATION

(11) **EP 4 161 954 B1**
(45) Date of publication and mention of the grant of the patent: **01.11.2023**
(21) Application number: 21745780.3
(22) Date of filing: 15.07.2021
(51) Int. Cl.: C07K 14/47, C07K 16/32, A61K 39/395

(54) **SERUM STABLE BINDING PROTEINS FOR HUMAN HER2 FOR THERANOSTIC APPLICATIONS**
SERUMSTABILE BINDUNGSPROTEINE FÜR MENSCHLICHES HER2 FÜR THERANOSTISCHE ANWENDUNGEN
PROTÉINES DE LIAISON SÉRIQUE STABLE POUR HER2 HUMAIN POUR APPLICATIONS THÉRANOSTIQUES

(30) Priority: 16.07.2020 EP 20186113; 04.02.2021 EP 21155334
(43) Date of publication of application: 12.04.2023
(73) Proprietor: NAVIGO PROTEINS GMBH, 06120 Halle/Saale (DE)
(72) Inventor: BOSSE-DOENECKE, Eva, 06120 Halle/Saale (DE); GLOSER-BRÄUNIG, Manja, 06120 Halle/Saale (DE); HAUPTS, Ulrich, 06120 Halle/Saale (DE)
(74) Representative: Haucke, Kerstin
(86) International application number: PCT/EP2021/069801
(87) International publication number: WO 2022/013376

(56) References cited:
- WO-A1-2016/124670
- WO-A1-2017/013129
- WO-A1-2017/013136
- WO-A1-2017/149002

## Description

### FIELD OF THE INVENTION

The present invention relates to new serum stable non-immunoglobulin binding proteins based on ubiquitin scaffold that are highly specific for membrane-bound receptor tyrosine kinase (Her2). The invention provides novel specific recombinant Her2 binding proteins with high serum stability essentially combined with high temperature stability and high affinity for Her2 for uses in medical applications for diagnosis or therapy of cancer with Her2 overexpression, in particular, for radiopharmaceutical applications.

### BACKGROUND OF THE INVENTION

Increased expression of the membrane-bound receptor tyrosine kinase Her2 plays an important role in the development and progression of many breast carcinomas, but also in ovarian, stomach, and uterine cancer, particularly with aggressive forms of cancer. Overexpression of this oncogene is reported for malignancies, predominantly in malignancies of epithelial origin, and is associated with cancer recurrence and poor prognosis. The three domain protein (extracellular, transmembrane, intracellular tyrosine kinase domain) is mediating cell proliferation and inhibiting apoptosis. Her2 forms dimerswhereby the intracellular domain of Her2 is activated which mediates cellular processes such as proliferation, differentiation, migration, or apoptosis. Thus, modulating the function of Her2 is an important approach for the development of cancer therapeutics, in particular those based on monoclonal antibodies binding to the extracellular domain of Her2. Therapeutic anti-Her2 monoclonal antibodies such as Trastuzumab or Pertuzumab are available for treatments of cancer, in particular breast cancer.

### Technical problems underlying the present invention

Known disadvantages of antibodies are a complex molecular structure, a large size, and challenging production methods. The large size of such molecules limits penetration of tumors having complex dense tissue structures. In addition, treatment of diseases with currently available Her2 binding molecules is not effective in all patients and may have severe side effects. Thus, a treatment with such antibodies is only possible after diagnosing the tumors as Her2-positive tumors. Tests on materials obtained by biopsie are variable and not precise.

Needless to say that there is a strong medical need to find novel agents that can be used in both diagnosis and therapy (theranostics) and overcome the disadvantages of currently available molecules.

It is thus an objective of the present invention to provide novel Her2 binding non-immunoglobulin molecules based on ubiquitin scaffold for new and improved strategies in the treatment and diagnosis of cancer with Her2 overexpression, in particular, for radiopharmaceutical applications. The objective is to provide specific recombinant binding proteins for a specific tumor indication that can be used both in diagnostic and therapy. In particular, it is an objective to provide novel binding proteins which have high affinity and specificity to Her2, combined with high structural stability in blood serum and at high temperatures.

A solution is provided in this invention by serum stable Her2 binding proteins (also known as Affilin^{®} molecules) as disclosed herein.The Her2 specific Affilin molecules of the invention are characterized by very high stability in serum and stability at high temperatures above 65 °C, and provide novel diagnostic and therapeutic options. Candidates for this application have to fullfill a combination of hallmarks to guarantee 1) efficient and specific isotopic labeling which requires reactions at higher temperatures; 2) stability in serum to enable sustained circulation and 3) high affinity for optimal targeting.

The invention provides novel optimized Her2 binding molecules for new and improved theranostics strategies for Her2 related cancer.

The above-described objectives and advantages are achieved by the subject-matters of the enclosed claims. The present invention meets the needs presented above by providing examples for Her2 binding proteins. The above overview does not necessarily describe all problems solved by the present invention. WO 2017/013129 describes Her2 binding proteins (affilins). The affinities for Her2 vary between 4.9 and 41.2nM and are stable at high temperatures.

### SUMMARY OF THE INVENTION

The present disclosure provides serum stable non-immunoglobulin binding proteins based on ubiquitin scaffold that are highly specific for membrane-bound receptor tyrosine kinase (Her2). The invention provides novel specific recombinant Her2 binding proteins with high serum stability essentially combined with high temperature stability and high affinity for Her2 for uses in medical applications for diagnosis or therapy of cancer with Her2 overexpression, in particular, for radiopharmaceutical applications. Provided are binding proteins for human Her2 comprising an amino acid sequence with at least 97 % identity to SEQ ID NO: 1, wherein the Her2 binding protein has a binding affinity for Her2 of less than 0.5 nM after 24 h incubation in serum as determined by ELISA, and is stable at temperatures of at least 64°C.

### BRIEF DESCRIPTION OF THE FIGURES

The Figures show:
**FIGURE 1** shows Her2 binding Affilin^{®} molecules with high stability in serum.
**FIGURES 2-7** show the stability of Her2 binding proteins in serum. The serum stability was determined via ELISA as described in Example 6.

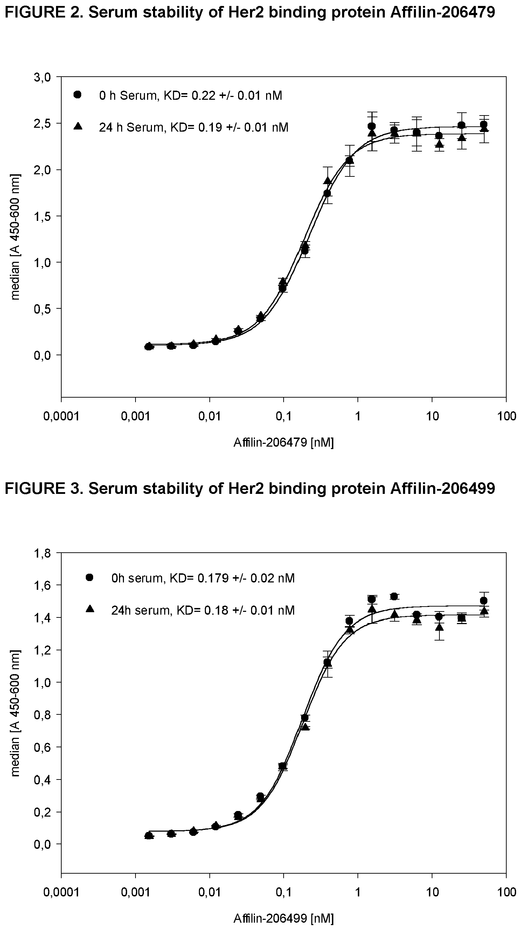

**FIGURE 2** shows the serum stability of Affilin-206479 (SEQ ID NO: 2)..
**FIGURE 3** shows the serum stability of Affilin-206499 (SEQ ID NO: 13).

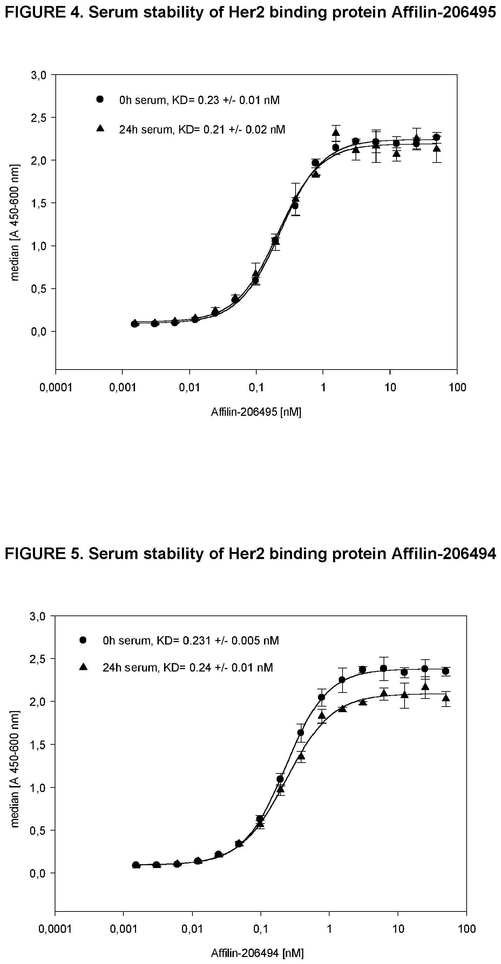

**FIGURE 4** shows the serum stability of Affilin-206494 (SEQ ID NO: 4).
**FIGURE 5** shows the serum stability of Affilin-206495 (SEQ ID NO: 10).

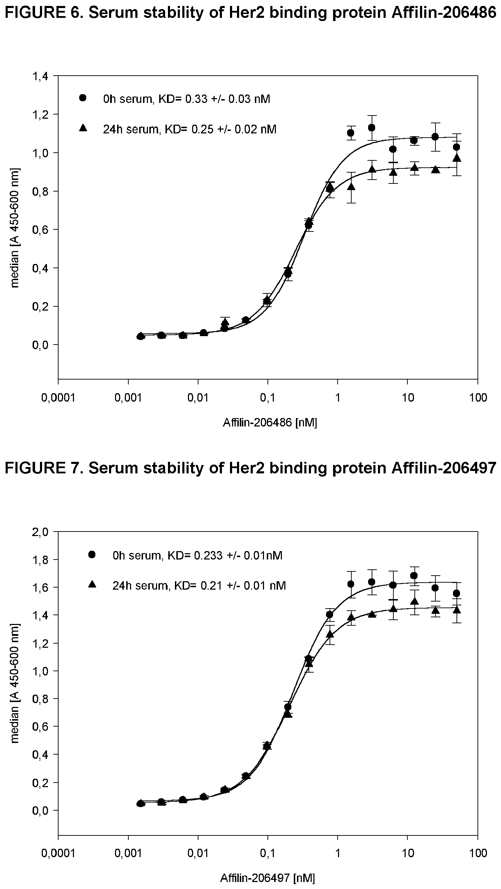

**FIGURE 6** shows the serum stability of Affilin-206486 (SEQ ID NO: 5).
**FIGURE 7** shows the serum stability of Affilin-206497 (SEQ ID NO: 14).

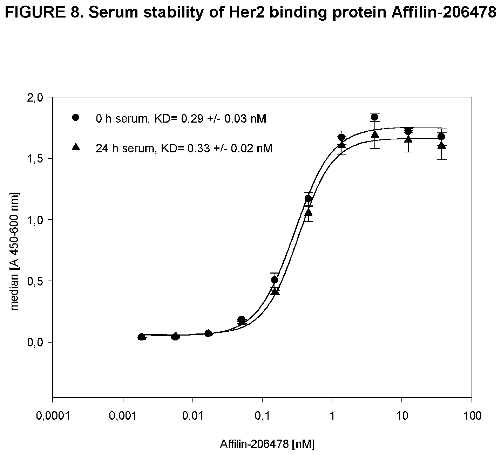

**FIGURE 8** shows the serum stability of Affilin-206478 (SEQ ID NO: 1).

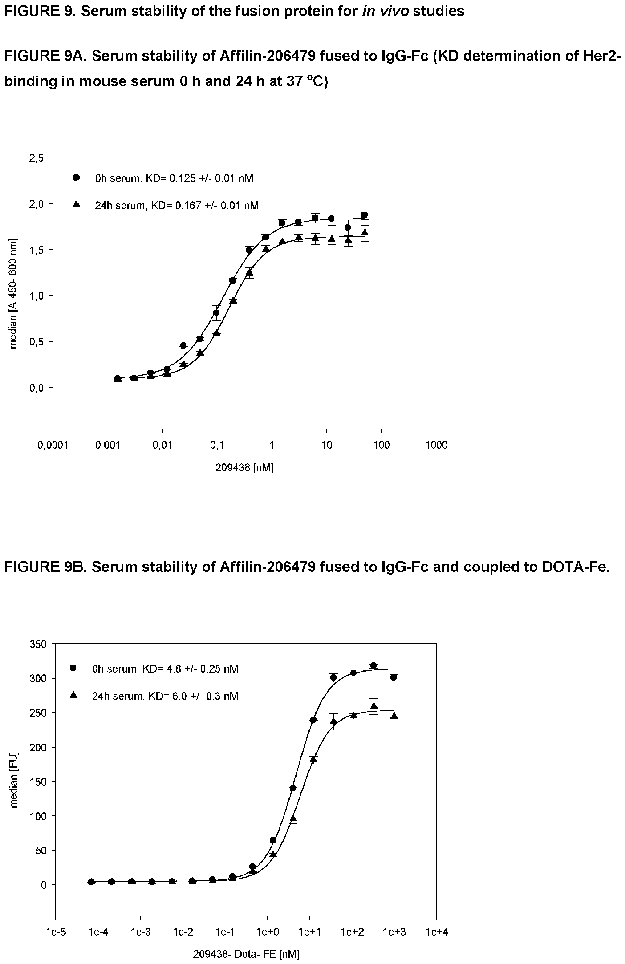

**FIGURE 9** shows the serum stability of the fusion protein Affilin-206479, in **FIGURE 9A** fused to murine IgG₂-Fc, in **FIGURE 9B** coupled to DOTA-Fe for *in vivo* studies.

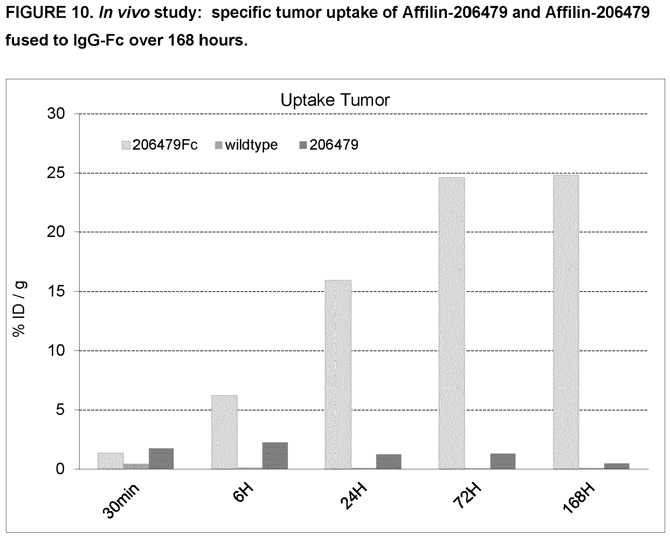

**FIGURE 10** shows the uptake of Affilin-206479 in percent injected dose per gram tumor tissue over seven days. Affilin-206479 was applied in unmodified form and as fusion with murine IgG₂-Fc in comparison to ubiquitin dimer control (SEQ ID NO: 16).

### DETAILED DESCRIPTION OF THE INVENTION

The present inventors have developed a solution to meet the strong ongoing need in the art for expanding medical options for the diagnosis and treatment of cancer by providing novel Her2 binding proteins. The Her2 specific proteins as defined herein are functionally characterized by high specific affinity for Her2. In particular, the invention provides Her2 binding proteins based on ubiquitin muteins (also known as Affilin^{®} molecules). The Her2 binding proteins as described herein provide molecular formats with favorable physicochemical properties, high-level expression in bacteria, and allow easy production methods. The novel Her2 binding proteins may broaden so far unmet medical strategies for the diagnosis and therapy of Her2 related cancer. In particular, the Her2 binding proteins may be used for diagnostic or imaging purposes, for example, for the presence of tumor cells expressing Her2, and for radiotherapy treatment of tumors expressing Her2. The new proteins are engineered to enable all relevant steps for a successful medical use.

Before the present invention is described in more detail below, it is to be understood that this invention is not limited to the particular methodology, protocols and reagents described herein as these may vary. It is also to be understood that the terminology used herein is for the purpose of describing particular aspects and embodiments only and is not intended to limit the scope of the present invention which is reflected by the appended claims. Unless defined otherwise, all technical and scientific terms used herein have the same meaning as commonly understood by one of ordinary skill in the art to which this invention belongs. This includes a skilled person working in the field of protein engineering and purification, but also including a skilled person working in the field of developing new target-specific binding molecules for use in technical applications and in therapy and diagnostics.

Preferably, the terms used herein are defined as described in A multilingual glossary of biotechnological terms: (IUPAC Recommendations)", Leuenberger, H.G.W, Nagel, B. and Kölbl, H. eds. (1995), Helvetica Chimica Acta, CH-4010 Basel, Switzerland).

Throughout this specification and the claims, which follow, unless the context requires otherwise, the word "comprise", and variants such as "comprises" and "comprising", was understood to imply the inclusion of a stated integer or step, or group of integers or steps, but not the exclusion of any other integer or step or group of integers or steps. The term "comprise(s)" or "comprising" may encompass a limitation to "consists" or "consisting of", should such a limitation be necessary for any reason and to any extent.

Several documents (for example: patents, patent applications, scientific publications, manufacturer's specifications, instructions, GenBank Accession Number sequence submissions etc.) may be cited throughout the present specification. Nothing herein is to be construed as an admission that the invention is not entitled to antedate such disclosure by virtue of prior invention. Some of the documents cited herein may be characterized as being *"incorporated by reference".* In the event of a conflict between the definitions or teachings of such incorporated references and definitions or teachings recited in the present specification, the text of the present specification takes precedence.

All sequences referred to herein are disclosed in the attached sequence listing that, with its whole content and disclosure, forms part of the disclosure content of the present specification.

### GENERAL DEFINITIONS

The term "Her2" refers to human Epidermal Growth Factor Receptor 2; synonym names are ErbB-2, Neu, CD340 orp185). Human Her2 is represented by the NCBI accession number NP_004439; the extracellular domain (residues 1-652) of Her2 is represented by the uniprot Accession Number p04626. The term "Her2" comprises all polypeptides which show a sequence identity of at least 70 %, 80 %, 85 %, 90 %, 95 %, 96 % or 97 % or more, or 100 % to NP_004439 and have the functionality of Her2.

The terms "Her2 binding protein" or "binding protein for human Her2" may be used herein interchangeably and refer to a protein with affinity binding to Her2.

The terms "protein" and "polypeptide" refer to any chain of two or more amino acids linked by peptide bonds, and does not refer to a specific length of the product. Thus, peptides, protein, amino acid chain, or any other term used to refer to a chain of two or more amino acids, are included within the definition of polypeptide", and the term polypeptide may be used instead of, or interchangeably with, any of these terms. The term polypeptide is also intended to refer to the products of post-translational modifications of the polypeptide, which are well known in the art. The term "modification" or "amino acid modification" refers to a substitution, a deletion, or an insertion of an reference amino acid at a particular position in a parent polypeptide sequence by another amino acid. Given the known genetic code, and recombinant and synthetic DNA techniques, the skilled scientist can readily construct DNAs encoding the amino acid variants. The term "Affilin" or "Affilin^{®}" (registered trademark of Navigo Proteins GmbH) refers to non-immunoglobulin derived binding proteins.

The term "substitution" is understood as exchange of an amino acid by another amino acid.

The terms binding "affinity" and "binding activity" may be used herein interchangeably, and they refer to the ability of a polypeptide to bind to another protein, peptide, or fragment or domain thereof. Binding affinity is typically measured and reported by the equilibrium dissociation constant (K_{D}), which is used to evaluate and rank order strengths of bimolecular interactions. The term "maximal binding" is expressed by Bₘₐₓ which is defining the experimental estimate of the maximum binding capacity at equilibrium of the binding event.

The term "fusion protein" relates to a protein comprising at least a first protein joined genetically to at least a second protein. A fusion protein is created through joining of two or more genes that originally coded for separate proteins. Fusion proteins may further comprise additional domains that are not involved in binding of the target, such as but not limited to, for example, multimerization moieties, polypeptide tags, polypeptide linkers or moieties binding to a target different from Her2.

The term "amino acid sequence identity" refers to a quantitative comparison of the identity (or differences) of the amino acid sequences of two or more proteins. Percent (%) amino acid sequence identity" with respect to a reference polypeptide sequence is defined as the percentage of amino acid residues in a sequence that are identical with the amino acid residues in the reference polypeptide sequence, after aligning the sequences and introducing gaps, if necessary, to achieve the maximum percent sequence identity. To determine the sequence identity, the sequence of a query protein is aligned to the sequence of a reference protein or polypeptide. Methods for sequence alignment are well known in the art. For example, for determining the extent of an amino acid sequence identity of an arbitrary polypeptide relative to the amino acid sequence of the SIM Local similarity program as known in the art is preferably employed. For multiple alignment analysis, Clustal Omega is preferably used, as known to someone skilled in the art.

### DETAILED DESCRIPTION OF THE EMBODIMENTS OF THIS INVENTION

The present invention will now be further described. In the following passages different embodiments of the invention are defined in more detail. Each embodiment defined below may be combined with any other embodiments unless clearly indicated to the contrary. In particular, any feature indicated as being preferred or advantageous may be combined with any other feature or features indicated as being preferred or advantageous.

### Structural characterization:

The Her2 binding protein of the invention comprises the amino acid sequence of 152 amino acids (SEQ ID NO: 15):

MQIFVKTLTGKTITLEVEPSDTTENVKAKIQDKEGIPPDQQTLAFVGKQLEDGRTLSDYNIQKES
TLWLYLTX1X2AAMRIFVX3THTGKTITLDVEPSDTIENVKAKIQDKEGIPPDQQRLIWAGKQLEDG

RTLSDYNIX₄EWX₅ILHLVLRLRAA wherein X₁ is selected from L or W, X₂ is selected from R or Y, X₃ is selected from K or T, X₄ is selected from Q or S, X₅ is selected from A or S.

In one embodiment, the Her2 binding protein is comprising an amino acid sequence of an amino acid sequence with at least 97 % amino acid sequence identity or at least 98 % amino acid sequence identity to any one selected from the group of SEQ ID NO: 1 (Affilin-206478), SEQ ID NO: 2 (Affilin-206479), SEQ ID NO: 3 (Affilin-206480), SEQ ID NO: 4 (Affilin-206494), SEQ ID NO: 5 (Affilin-206486), or SEQ ID NO: 6 (Affilin-206482). In one embodiment, the Her2 binding protein is comprising an amino acid sequence of an amino acid sequence with at least 97 %, 98 %, 99 % or 100 % amino acid sequence identity to SEQ ID NO: 1 (Affilin-206478). In one embodiment, the Her2 binding protein is comprising an amino acid sequence of an amino acid sequence with at least 97 %, 98 %, 99 % or 100 % amino acid sequence identity to SEQ ID NO: 2 (Affilin-206479). In some embondiments, the Her2 binding protein comprises an amino acid sequence of any of amino acid sequences as shown in SEQ ID NOs: 1-14. The present invention surprisingly shows that the Her2 binding proteins of the invention maintain 2, 3, 4, or 5 amino acid residues of wild-type positions L73, R74, K82, Q138, or S141 of SEQ ID NO: 16 (di-ubiquitin).

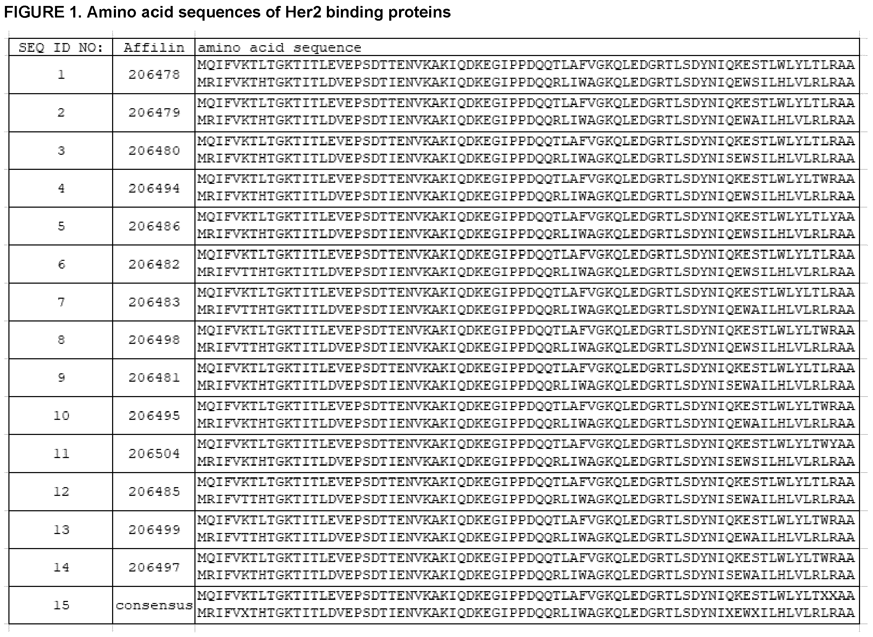

In some embodiments, examples for stable Her2 binding proteins comprise or consist of an amino acid sequence selected from any of SEQ ID NOs: 1-14, as shown in **FIGURE 1****,** or of amino acid sequences with at least 97 % identity to any of SEQ ID NOs: 1-14. In some embodiments, examples for stable Her2 binding proteins comprise or consist of an amino acid sequence selected from any of SEQ ID NOs: 1-14, as shown in **FIGURE 1****,** or of amino acid sequences with at least 98 % identity to any of of SEQ ID NOs: 1-14. In various embodiments, the Her2 binding proteins comprises an amino acid sequence selected from SEQ ID NO: 1 or SEQ ID NO: 2, or of amino acid sequences with at least 97 % identity to any of SEQ ID NO: 1 or SEQ ID NO: 2. In various embodiments, the Her2 binding proteins comprises an amino acid sequence selected from SEQ ID NO: 1 or SEQ ID NO: 2, or of amino acid sequences with at least 98 % identity to any of of SEQ ID NO: 1 or SEQ ID NO: 2.

In some embodiments, the binding protein for Her2 has at least 97 %, 98 %, 99 %, or 100 % sequence identity to any one of the amino sequences of SEQ ID NOs: 1-14. In some embodiments, the binding protein for Her2 has at least 97 %, 98 %, 99 %, or 100 % sequence identity to SEQ ID NO: 1 or SEQ ID NO: 2. In various other embodiments described herein referring to at least 97 % sequence identity, the sequence identity may preferably be any of at least 97 %, 98 %, 99 %, or 100 % identity to the respective reference sequence.

In some embodiments, the stable Her2 binding protein as disclosed herein may have any amino acid identity of at least 90.5 % identity to the amino acid sequence of bis-ubiquitin (SEQ ID NO: 16).

### Functional characterization.

The Her2 binding protein of the invention is functionally characterized by a high stability even in serum. Even after long incubation in blood serum, the binding protein has not more than threefold or twofold loss in binding affinity for Her2, compared to the binding affinity before incubation in serum (see Examples). Preferably, even after long incubation in blood serum, the binding protein has at least 85 % of the maximal binding for Her2 expressed as Bₘₐₓ and not more than threefold or twofold loss in binding affinity for Her2, compared to the binding affinity before incubation in serum (see Examples). The binding affinity even after 24 h in blood serum is below 0.5 nM, preferably as determined by ELISA. Combined with extremely high stability in serum, reflected by the maximum binding and high affinity to Her2, the Her2 binding proteins are stable at temperatures of at least 64 °C, preferable in the range of 64 °C up to 70 °C. The surprising combination of several functional features, namely the extrem high stability in serum, the high temperature stability and high affinity to Her2 even in serum, is making the proteins of the invention suitable for a use in medical applications, both diagnostic and therapeutic applications. The binding protein for human Her2 as disclosed herein comprises an amino acid sequence selected from the group of any of SEQ ID NOs: 1-14 wherein the binding protein for human Her2
(i) has not more than 3fold loss in binding affinity (KD) for Her2 after incubation for at least 24 h in serum, as compared to the binding affinity before incubation in serum, and
(ii) has at least 85 % of the maximal binding (Bmax) for Her2 after incubation for at least 24 h in serum, and
(iii) has a binding affinity for Her2 of less than 0.5 nM after 24 h incubation in serum, as determined by ELISA, and
(iv) is stable at temperatures of at least 64 °C.

In some embodiments, the Her2 binding protein as described herein binds to Her2 expressed on cells and/or has a binding affinity to Her2 of 5 nM or less, preferably between 0.1 - 2 nM, preferably less than 0.5 nM even if incubated for 24 h in serum, for example in mouse serum.

In some embodiments, the Her2 binding protein as described herein has a binding affinity (K_{D}) of less than 5 nM for Her2, as determined after incubation for 24 h in serum. The Her2 binding proteins bind Her2 with measurable binding affinity of less than 5 nM, less than 3 nM, and less than 2 nM, and less than 0.5 nM as determined after incubation for 24 h in mouse serum. The appropriate methods are known to those skilled in the art or described in the literature. The methods for determining the binding affinities are known *per se* and can be selected for instance from the following methods known in the art: enzyme-linked immunosorbent assay (ELISA), surface plasmon resonance (SPR), kinetic exclusion analysis (KinExA assay), Bio-layer interferometry (BLI), flow cytometry, fluorescence spectroscopy techniques, isothermal titration calorimetry (ITC), analytical ultracentrifugation, radioimmunoassay (RIA or IRMA), and enhanced chemiluminescence (ECL). Some of the methods are described in the Examples below. Typically, the dissociation constant K_{D} is determined at the range of temperatures between 20 °C and 30 °C, for example at 20 °C, 25 °C, or 30 °C. The lower the K_{D} value, the greater the binding affinity of the biomolecule for its binding partner. The higher the K_{D} value, the weaker the binding partners bind to each other. The Her2 binding protein as described herein binds to Her2 but does not detectably bind to human Fc-domain of immunoglobulin IgG₁, as determined by surface plasmon resonance assays. A binding to Her2 with K_{D} less than 5 nM, preferably less than 0.5 nM, may be important for targeted medical applications for Her2 related cancer. Further, a protein with Her2 binding with K_{D} less than 5 nM, preferably less than 0.5 nM, may have reduced potential toxic side effects.

In some embodiments, the Her2 binding protein as described herein is stable at high temperatures, higher than 64 °C, preferably higher than 65 °C, preferably up to 70 °C, or even higher. Such high temperature stability of a Her2 binding protein is important for several applications, for example, for radiochemical applications. For stability analysis, for example spectroscopic or fluorescence-based methods in connection with chemical or physical unfolding are known to those skilled in the art. For example, the stability of a molecule can be determined by measuring the thermal melting (Tₘ) temperature, the temperature in °Celsius (°C) at which half of the molecules become unfolded, using standard methods. Typically, the higher the Tₘ, the more stable the molecule. Temperature stability was determined by differential scanning fluorimetry (DSF), as described in further detail in the **Examples.**

**Molecules with extended half-life.** For diagnostic and therapeutic applications, it might be of great importance to have molecules with extended half-life. Some embodiments relate to a Her2 binding protein of any one selected from SEQ ID NOs: 1-14, or at least 97 % or at least 98 % identical proteins thereto, preferably selected from a protein with at least 97 % identity to SEQ ID NO: 1 or SEQ ID NO: 2, fused to molecules which extend the half-life of the Her2 binding protein. Some embodiments relate to fusion proteins comprising stable Her2 binding proteins of SEQ ID NOs: 1-6 or at least 97 % identical proteins thereto, for example SEQ ID NOs: 1-14, as described herein. In various embodiments, Her2 binding proteins that comprise an amino acid sequence selected from SEQ ID NOs: 1 or SEQ ID NO: 2, or of amino acid sequences with at least 97 % identity to any of of SEQ ID NO: 1 or SEQ ID NO: 2, are fused to molecules that extend the half-life of the Her2 binding protein. In various embodiments, Her2 binding proteins that comprise an amino acid sequence with at least 98 % identity to any of of SEQ ID NO: 1 or SEQ ID NO: 2, are fused to molecules that extend the half-life of the Her2 binding protein. Some embodiments relate to fusion proteins comprising stable Her2 binding proteins of SEQ ID NO: 1 or SEQ ID NO: 2 or at least 97 %, 98 %, or 99 % identical proteins thereto.

The fusion proteins may comprise additionally at least one further molecule, in addition to the stable Her2 binding protein, **modulating pharmacokinetics.** A fusion protein comprising the stable Her2 binding protein as described herein and a molecule modulating pharmacokinetics might have an extended half-life. Suitable molecule for half-life extension can be selected from a serum albumin (e.g human serum albumin or mouse serum albumin), an albumin-binding protein, an immunoglobulin binding protein, or an immunoglobulin or immunoglobulin fragment, a polysaccharide (for example, hydroxylethyl starch), an unstructured amino acid sequence which increases the hydrodynamic radius such as a multimer comprising amino acids alanine, glycine, serine, proline, or a polyethylene glycol. An example for a fusion protein comprising a stable Her2 binding protein as described above fused to an immunoglobulin is given in SEQ ID NO: 18 and serum stability of such a fusion protein is shown in **FIGURE 9****.** In some embodiments, the fusion protein is comprising a Her2 binding protein as described herein at the N-terminus of the fusion protein and the immunoglobulin binding protein (as monomer, dimer, trimer, or tetramer) at the C-terminus.

Several techniques for producing Her2 binding protein with extended half-life are known in the art, for example, direct fusions of the moiety modulating pharmacokinetics with the Her2 binding protein as described above or chemical coupling methods. In some embodiments, the moiety modulating pharmacokinetics can be attached for example directly or at one or several sites of the Her2 binding protein through a peptide linker sequence or through a coupling site as described above.

**Further moieties.** In some embodiments, conjugation of proteinaceous or non-proteinaceous moieties to the Her2 binding protein may be performed applying chemical methods well-known in the art. In some embodiments, coupling chemistry specific for derivatization of cysteine or lysine residues may be applicable. Chemical coupling can be performed by chemistry well known to someone skilled in the art, including but not limited to, substitution, addition or cycloaddition or oxidation chemistry (e.g. disulfide formation).

Some embodiments relate to a fusion protein wherein the fusion protein comprises additionally at least one least one **diagnostically active moiety.** Such diagnostically active moiety might be selected from a radionuclide, fluorescent protein, photosensitizer, dye, or enzyme, or any combination of the above, In various embodiments, the Her2 binding protein further comprises a diagnostic moiety. In other embodiments, the Her2 binding protein further comprises more than one diagnostic moiety. In some embodiments, a Her2 binding protein that comprises at least one diagnostic moiety can be employed, for example, as imaging agent, for example to evaluate presence of tumor cells or metastases, tumor distribution, and/or recurrence of tumor. Methods for detection or monitoring of cancer cells involve imaging methods. Such methods involve imaging Her2 related cancer cells by, for example, radioimaging or photoluminescens or fluorescence. Some embodiments refer to methods of imaging at least a portion of a subject comprising administering a composition to the subject, wherein the composition comprises an isolated polypeptide of SEQ ID NOs: 1-14, preferably of a protein with at least 97 %, 98 %, 99 %, or even 100 % identity to SEQ ID NO: 1 or SEQ ID NO: 2, and an imaging agent. In some embodiments the method is further comprising the step of diagnosing the subject with a Her2 expressing cancer, for example, breast cancer. In some embodiments the method is further comprising the step of monitoring the delivery of the composition to the subject.

Some embodiments relate to a fusion protein wherein the fusion protein comprises in addition to the Her2 specific protein as described above at least one **therapeutically active moiety.** Such therapeutically active moiety can be selected from a monoclonal antibody or a fragment thereof, a binding protein, a receptor or receptor domain, a radionuclide, a cytotoxic compound, a cytokine, a chemokine, an enzyme, or derivatives thereof, or any combination of the above. In some embodiments, the Her2 binding protein that comprises a therapeutically active component may be used in targeted delivery of any of the above listed components to the Her2 expressing tumor cell and accumulate therein, thereby resulting in low levels of toxicity to normal cells. In some embodiments, fusion proteins comprising a stable Her2 bindng protein as described herein and an monomclonal antibody are referred to as Mabfilin^{™}.

Suitable **radionuclides** for applications in imaging *in vivo or in vitro* or for radiotherapy include for example but are not limited to the group of gamma-emitting isotopes, the group of positron emitters, the group of beta-emitters, and the group of alpha-emitters. In some embodiments, suitable conjugation partners include chelators such as 1,4,7,10-tetraazacyclododecane-1,4,7,10-tetraacetic acid (DOTA) or diethylene triamine pentaacetic acid (DTPA) or their activated derivatives, nanoparticles and liposomes. In various embodiments, DOTA may be suitable as complexing agent for radioisotopes and other agents for imaging, as described in the **Examples** in further detail. Some embodiments refer to methods of imaging at least a portion of a subject comprising administering a composition to the subject, wherein the composition comprises an isolated polypeptide of SEQ ID NOs: 1-14, preferably of a protein with at least 97 %, 98 %, 99 %, or even 100 % identity to SEQ ID NO: 1 or SEQ ID NO: 2, and a radionuclide conjugated to a suitable conjugation partner, for example, DOTA. In some embodiments the method is further comprising the step of diagnosing the subject with a Her2 expressing cancer, for example, breast cancer. In some embodiments the method is further comprising the step of monitoring the delivery of the composition to the subject.

In some embodiments, additional amino acids can extend either at the N-terminal end of the Her2 binding protein or the C-terminal end or both. Additional sequences may include for example sequences introduced e.g. for purification or detection. In one embodiment, additional amino acid sequences include one or more peptide sequences that confer an affinity to certain chromatography column materials. Typical examples for such sequences include, without being limiting, Strep-tags, oligohistidine-tags, glutathione S-transferase, maltose-binding protein, inteins, intein fragments, or the albumin-binding domain of protein G. Any references in the description to methods of treatment refer to the compounds, pharmaceutical compositions and medicaments of the present invention for use in a method for treatment of the human (or animal) body by therapy (or for diagnosis)

**Use in medicine.** Various embodiments relate to the Her2 binding protein as disclosed herein for use in medicine. In one embodiment, the Her2 binding protein is used in medicine to diagnose or treat cancer associated with Her2 expression. The Her2 binding proteins as disclosed herein allow selective **diagnosis and treatment** of Her2 related cancer cells or cancer tissues. Her2 is known to be upregulated in tumor cells. The membrane protein Her2 is known to be upregulated in tumor cells, resulting in uncontrolled growth of tumor cells and in the formation of metastases. New therapies for cancer patients include an inhibition the growth of Her2 expressing cells by targeted therapeutics such as for example the monoclonal antibodies Trastuzumab (Herceptin^{®}) or Pertuzumab (Perjeta^{®}). T-DM1, an antibody-drug conjugate, is highly effective against breast, uterine, and ovarian carcinosarcoma overexpressing Her2.

Overexpression of Her2 has been described in a wide variety of cancers. For example, overexpression of Her2 occurs in approximately 15 % to 30 % of breast cancers and 10 % to 30 % of gastric/gastroesophageal cancers, and has also been observed in other cancers like ovary, endometrium, bladder, lung colon, head and neck. Thus, the pharmaceutical composition comprising the Her2 binding protein as described herein, can be used for treatment of cancer in which Her2 is relevant for the development of the disease including but not limited to particularly breast, ovarian, gastric, but also in lung, head and neck, cervical, prostate, pancreas, and others. One embodiment is a method of diagnosing (including monitoring) a subject having Her2 related cancer, the method of diagnosis (monitoring) comprising administering to the subject the Her2 binding protein as described, optionally conjugated to radioactive molecules. In various embodiments, the Her2 binding protein as disclosed herein may be used for diagnosis of Her2 related cancer, optionally wherein the Her2 binding protein is conjugated to a radioactive molecule. In some embodiments, imaging methods using the Her2 binding protein with labels such as radioactive or fluorescent can be employed to visualize Her2 on specific tissues or cells, for example, to evaluate presence of Her2 related tumor cells, Her2 related tumor distribution, recurrence of Her2 related tumor, and/or to evaluate the response of a patient to a therapeutic treatment.

One embodiment is a method of treating a subject having Her2 related cancer, the method of treatment comprising administering to the subject the Her2 specific binding protein as described, optionally conjugated to a radioactive molecule and/or a cytotoxic agent. In various embodiments, the Her2 binding protein as disclosed herein may be used for treatment of Her2 related cancer, optionally wherein the Her2 binding protein is conjugated to a cytotoxic agent and/or to a radioactive molecule. Some embodiments relate to the use of the Her2 binding protein labelled with a suitable radioisotope or cytotoxic compound for treatment of Her2 related tumor cells, in particular to control or kill Her2 related tumor cells, for example malignant cells. In one embodiment, curative doses of radiation are selectively delivered to Her2 related tumor cells but not to normal cells.

**Compositions.** Various embodiments relate to a composition comprising the Her2 binding protein as disclosed herein. Some embodiments relate to a composition comprising the Her2 binding protein as defined above for use in medicine, preferably for use in the diagnosis or treatment of various Her2 related cancer tumors as described above. Compositions comprising the Her2 binding protein as described above may be used for clinical applications for both diagnostic and therapeutic purposes. In particular, compositions comprising the Her2 binding protein as described herein may be used for clinical applications for imaging, monitoring, and eliminating or inactivating pathological cells that express Her2.

Various embodiments relate to a diagnostic composition for the diagnosis of Her2 related cancer comprising the Her2 binding protein as defined herein and a diagnostically acceptable carrier and/or diluent. These include for example but are not limited to stabilizing agents, surface-active agents, salts, buffers, coloring agents etc. The compositions can be in the form of a liquid preparation, a lyophilisate, granules, in the form of an emulsion or a liposomal preparation.

The diagnostic composition comprising the Her2 binding protein as described herein can be used for diagnosis of Her2 related cancer, as described above.

Various embodiments relate to a pharmaceutical (e.g. therapeutical) composition for the treatment of diseases comprising the Her2 binding protein as disclosed herein, and a pharmaceutically (e.g. therapeutically) acceptable carrier and/or diluent. The pharmaceutical (e.g. therapeutical) composition optionally may contain further auxiliary agents and excipients known *per se.* These include for example but are not limited to stabilizing agents, surface-active agents, salts, buffers, coloring agents etc.

The pharmaceutical composition comprising the Her2 binding protein as defined herein can be used for treatment of diseases, as described above.

The compositions contain an effective dose of the Her2 binding protein as defined herein. The amount of protein to be administered depends on the organism, the type of disease, the age and weight of the patient and further factors known *per se.* Depending on the galenic preparation these compositions can be administered parentally by injection or infusion, systemically, intraperitoneally, intramuscularly, subcutaneously, transdermally, or by other conventionally employed methods of application.

The composition can be in the form of a liquid preparation, a lyophilisate, a cream, a lotion for topical application, an aerosol, in the form of powders, granules, in the form of an emulsion or a liposomal preparation. The type of preparation depends on the type of disease, the route of administration, the severity of the disease, the patient and other factors known to those skilled in the art of medicine.

The various components of the composition may be packaged as a kit with instructions for use.

**Preparation of Her2 binding proteins.** Her2 binding proteins as described herein may be prepared by any of the many conventional and well known techniques such as plain organic synthetic strategies, solid phase-assisted synthesis techniques, fragment ligation techniques or by commercially available automated synthesizers. On the other hand, they may also be prepared by conventional recombinant techniques alone or in combination with conventional synthetic techniques. Furthermore, they may also be prepared by cell-free *in vitro* transcription/translation. Various embodiments relate to a **polynucleotide** encoding a Her2 binding protein as disclosed herein. One embodiment further provides an expression **vector** comprising said polynucleotide, and a host cell comprising said isolated polynucleotide or the expression vector.

Various embodiments relate to a **method for the production** of a Her2 binding protein as disclosed herein comprising culturing of a host cell under suitable conditions which allow expression of said Her2 binding protein and optionally isolating said Her2 binding protein.

For example, one or more polynucleotides which encode for the Her2 binding protein may be expressed in a suitable host and the produced Her2 binding protein can be isolated. A host cell comprises said nucleic acid molecule or vector. Suitable host cells include prokaryotes or eukaryotes. A vector means any molecule or entity (e.g., nucleic acid, plasmid, bacteriophage or virus) that can be used to transfer protein coding information into a host cell. Various cell culture systems, for example but not limited to mammalian, yeast, plant, or insect, can also be employed to express recombinant proteins. Suitable conditions for culturing prokaryotic or eukaryotic host cells are well known to the person skilled in the art. Cultivation of cells and protein expression for the purpose of protein production can be performed at any scale, starting from small volume shaker flasks to large fermenters, applying technologies well-known to any skilled in the art. One embodiment is directed to a method for the preparation of a binding protein as detailed above, said method comprising the following steps: (a) preparing a nucleic acid encoding a Her2 binding protein as defined herein; (b) introducing said nucleic acid into an expression vector; (c) introducing said expression vector into a host cell; (d) cultivating the host cell; (e) subjecting the host cell to culturing conditions under which a Her2 binding protein is expressed, thereby producing a Her2 binding protein as defined herein; (f) optionally isolating the Her2 binding protein produced in step (e); and (g) optionally conjugating the Her2 binding protein with further functional moieties as defined herein.

In general, isolation of purified Her2 binding protein from the cultivation mixture can be performed applying conventional methods and technologies well known in the art, such as centrifugation, precipitation, flocculation, different embodiments of chromatography, filtration, dialysis, concentration and combinations thereof, and others. Chromatographic methods are well-known in the art and comprise without limitation ion exchange chromatography, gel filtration chromatography (size exclusion chromatography), hydrophobic interaction chromatography, or affinity chromatography.

For simplified purification, the Her2 binding protein can be fused to other peptide sequences having an increased affinity to separation materials. Preferably, such fusions are selected that do not have a detrimental effect on the functionality of the Her2 binding protein or can be separated after the purification due to the introduction of specific protease cleavage sites. Such methods are also known to those skilled in the art.

### EXAMPLES

The following Examples are provided for further illustration of the invention. The invention is particularly exemplified by a maximum of 14 modifications of a ubiquitin dimer resulting in binding to Her2. The following Examples merely show the practicability of the invention on the basis of the above description.

### Example 1. Her2 binding proteins

The sequence of the ubiquitin dimer is shown in SEQ ID NO: 16: 
MQIFVKTLTGKTITLEVEPSDTIENVKAKIQDKEGIPPDQQRLIFAGKQLEDGRTLSDYNIQKEST
LHLVLRLRAAMQIFVKTLTGKTITLEVEPSDTIENVKAKIQDKEGIPPDQQRLIWAGKQLEDGRTL
SDYNIQKESTLHLVLRLRAA.
 13 substitutions in amino acid sequence as shown in SEQ ID NO: 16 to generate an Her2 binding protein are as follows:
I23T, R42T, I44A, A46V, H68W, V70Y, R72T, Q78R, L84H, E92D, K139E, E140W, and T142I.

The resulting Her2 binding protein has the amino acid sequence of 152 amino acids (modified amino acids are underlined; modified as compared to SEQ ID NO: 16):

MQIFVKTLTGKTITLEVEPSDTTENVKAKIQDKEGIPPDQQTLAFVGKQLEDGRTLSDYNIQKES
TLWLYLTLRAAMRIFVKTHTGKTITLDVEPSDTIENVKAKIQDKEGIPPDQQRLIWAGKQLEDGR
TLSDYNIQEWSILHLVLRLRAA (SEQ ID NO: 1).

The Her2 binding protein as described herein have at least 97 % identity to SEQ ID NO: 15 or to SEQ ID NO: 1.

Optionally, further substitutions comprise at least one of amino acids L73W or R74Y or K82T or Q138S or S141A in the ubiquitin dimer (SEQ ID NO: 16). The resulting Her2 binding protein may have between 90.5 % amino acid sequence identity and 92 % amino acid sequence identity to the ubiquitin dimer of SEQ ID NO: 16. The higher the amino acid identity to the ubiquitin dimer, the more stable is the novel binding protein, as shown below.

Examples for novel Her2 binding proteins with surprising high stability are shown in SEQ ID Nos: 1-14.

### Example 2. Expression and purification of Her2 binding proteins

Affilin^{®} molecules were cloned into an expression vector using standard methods known to a skilled person, purified and analyzed as described below. All Affilin proteins were expressed in *E. coli* and highly purified by affinity chromatography and gel filtration.

BL21 (DE3) competent cells were transformed with an expression plasmid encoding the respective Affilin protein. Cells were spread onto selective agar plates (Kanamycin) and incubated either for 2 days at 21 °C or overnight at 37 °C. Precultures were inoculated from single colony in 3 ml 2xYT medium supplemented with 50 µg/ml kanamycin and cultured for 6 hours at 37 °C at 210 rpm in a conventional orbital shaker in 14 mL culture tubes. For main cultures 350 mL ZYM-5052 medium (see Studier 2005) were inoculated with entire precultures and incubated in 2.5 L Ultra Yield^{™} flasks at 30 °C at 230 rpm in an orbital shaker. The culture medium was supplemented with 50 µg/ml Kanamycin and antifoam SE15. Recombinant protein expression was induced by metabolizing glucose and subsequently allowing lactose to enter the cells. Cells were grown overnight for approx. 18 hours to reach a final OD₆₀₀ of about 10-20. Before the harvest, the OD₆₀₀ was measured, samples adjusted to 0.6/OD₆₀₀ were withdrawn, pelleted and frozen at -20 °C. To collect biomass cells were centrifuged at 12000 x g for 20 min at 20 °C. Pellets were weighed (wet weight) and stored at -20 °C before processing.

Proteins with affinity tag were purified by affinity chromatography and size exclusion. After affinity chromatography purification using Strep-Tactin Superflow high capacity cartridges (IBA Lifesciences) a size exclusion chromatography (SEC) has been performed using an ÄKTA system and a Superdex^{™} 75 HiLoad 16/600 column (GE Healthcare). Homogenous species of all purified ligands were obtained. Following SDS-PAGE analysis positive fractions were pooled and their protein concentrations were measured.

Further analysis included SDS-PAGE, SE-HPLC and RP-HPLC. Protein concentrations were determined by absorbance measurement at 280 nm using the molar absorbent coefficient. RP chromatography (RP HPLC) has been performed using a Dionex HPLC system and a Vydac 214MS54 C4 (4.6 × 250 mm, 5 µm, 300 Å) column (GE Healthcare).

**Mammalian expression of Her2-mlgG-Fc-fusion-proteins.** Expi293-F-cells were cultured with 0.5- 1 Mio cells/ml in Expi293-F Expression medium (Fisher scientific, 13489756) in shake flasks with 135 rpm, at 37 °C, 8 % CO2 and 95 % humidity. 1 day before transfection, cells were seeded with a density of 2.0 Mio/ cells/ ml. On the day of transfection, cells were seeded with a density of 2.5 Mio/ ml. 1 µg plasmid- DNA of fusion protein (Affilin-206479 fused to murine IgG₂-Fc; SEQ ID NO: 18) per ml of culture volume were used and diluted in Opti-MEM^{®} I Reduced Serum Medium (Life Technologies, 31985-062). ExpiFectamine^{™} was diluted in Opti-MEM^{®} I Reduced Serum Medium, according to manufacturer information and incubated for 5 min at rt. Subsequently, DNA-solution was added to the ExpiFectamine-mixture and incubated for 20 min at rt, before it was given to the cells. After 16 h enhancer was added to the cells. Supernatant was collected after 96- 120 h, centrifuged and filtered through 0.45 µm membrane.

### Example 3. Solubility analysis of Her2 binding proteins

Samples were resuspended in 90 µl extraction buffer (PBS supplemented with 0.5x BugBuster, 6 mM MgSO₄, 6 mM MgCl₂, 15 U/mL Benzonase) and solubilized by agitation in a thermomixer at 850 rpm, rt for 15 min with a subsequent incubation at -80 °C for 15 min. After thawing, soluble proteins were separated from insoluble proteins by centrifugation (16000 x g, 2 min, rt). Supernatant was withdrawn (soluble fraction) and the pellet (insoluble fraction) was resuspended in equivalent amount of urea buffer (8 M urea, 0.2 M Tris, 20 mM EDTA, pH 7.0). 35 µl were taken both from the soluble and insoluble fraction, and 10 µl 5x sample buffer as well as 5 µl 0.5 M DTT were added. Samples were boiled at 95 °C for 5 min. Finally, 5 µl of those samples were applied to NuPage Novex 4-12 % Bis-Tris SDS gels which were run in accordance to the manufacturer's recommendations and stained with Coomassie. Results are shown in **Table 1.**

**Table 1. Her2 binding proteins - expression, purification, solubiltity.**

| | | solubility | yield per volume | yield per weight | rp-HPLC |
|---|---|---|---|---|---|
| SEQ ID NO: | Affilin- | Soluble expression | Yield per L | Yield per g | main peak |
| 1 | 206478 | 80 % | 35.15 mg/L | 1.62 mg/g | 92.03 % |
| 2 | 206479 | 80 % | 21.04 mg/L | 1.08 mg/g | 93.67 % |
| 3 | 206480 | 80 % | 38.28 mg/L | 1.79 mg/g | 92.07 % |
| 4 | 206494 | 75 % | 54.93 mg/L | 2.67 mg/g | 92.51 % |
| 5 | 206486 | 75 % | 27.89 mg/L | 1.24 mg/g | 92.95 % |
| 6 | 206482 | 90 % | 62.40 mg/L | 2.99 mg/g | 96.96 % |
| 7 | 206483 | 90 % | 70.06 mg/L | 3.31 mg/g | 95.78 % |
| 8 | 206498 | 95 % | 45.92 mg/L | 2.30 mg/g | 95.97 % |
| 9 | 206481 | 80 % | 43.52 mg/L | 1.95 mg/g | 92.47 % |
| 10 | 206495 | 95 % | 38.04 mg/L | 1.99 mg/g | 94.05 % |
| 12 | 206485 | 90 % | 43.29 mg/L | 2.44 mg/g | 93.33 % |
| 13 | 206499 | 95 % | 75.09 mg/L | 3.60 mg/g | 94.49 % |
| 14 | 206497 | 95 % | 54.23 mg/L | 2.64 mg/g | 92.02 % |

### Example 4. Her2 binding proteins are stable at high temperatures

Thermal stability of the binding proteins of the invention was determined by Differential Scanning Fluorimetry (DSF). Each probe was transferred at concentrations of 0.1 µg/µL to a MicroAmp Optical 384-well plate well plate, and SYPRO Orange dye was added at suitable dilution. A temperature ramp from 25 to 95 °C was programmed with a heating rate of 1 °C per minute (ViiA-7 Applied Biosystems). Fluorescence was constantly measured at an excitation wavelength of 520 nm and the emission wavelength at 623 nm (ViiA-7, Applied Biosystems). **Results:** The midpoints of transition for the thermal unfolding (Tm, melting points) are shown for selected variants in **Table 2.** Her2 binding proteins of the invention have similar melting temperatures higher than 64 °C.

### Example 5. Analysis of Her2 binding proteins (Surface Plasmon Resonance, SPR)

A CM5 sensor chip (GE Healthcare) was equilibrated with SPR running buffer. Surface-exposed carboxylic groups were activated by passing a mixture of EDC and NHS to yield reactive ester groups. 700-1500 RU Her2-Fc(on-ligand) were immobilized on a flow cell, IgG-Fc (off- ligand) was immobilized on another flow cell at a ratio of 1:3 (hlgG-Fc:Target) to the target. Injection of ethanolamine after ligand immobilization was used to block unreacted NHS groups. Upon ligand binding, protein analyte was accumulated on the surface increasing the refractive index. This change in the refractive index was measured in real time and plotted as response or resonance units (RU) versus time. The analytes were applied to the chip in serial dilutions with a flow rate of 30 µl/min. The association was performed for 30 seconds and the dissociation for 60 seconds. After each run, the chip surface was regenerated with 30 µl regeneration buffer and equilibrated with running buffer. A dilution series of Trastuzumab served as positive control, whereas a dilution series of unmodified di-ubiquitin represents the negative control. The control samples were applied to the matrix with a flow rate of 30 µl/min, while they associate for 60 seconds and dissociate for 120 seconds. Regeneration and re-equilibration were performed as previously mentioned. Binding studies were carried out by the use of the Biacore 3000 (GE Healthcare); data evaluation was operated via the BIAevaluation 3.0 software, provided by the manufacturer, by the use of the Langmuir 1:1 model (RI=0). **Results** of binding to Her2 are shown in **Table 2.** Evaluated dissociation constants (K_{D}) were standardized against *off*-target and indicated. The column "amino acid" refers to the corresponding amino acid in the indicated position (e.g. 73, ,74, 82, 138, 141). The amino acids of SEQ ID NO: 1 in positions 73, 74, 82, 138, 141 are L, R, K, Q, S are identical to ubiquitin (dimer) (SEQ ID NO: 16). In SEQ ID NOs: 2-6, 1 position selected from positions 73, 74, 82, 138, 141 of SEQ ID NO: 1 is replaced by a different amino acid, as indicated by underline in Table 2. In SEQ ID NOs: 7-10, 2 positions selected from positions 73, 74, 82, 138, 141 of SEQ ID NO: 1 are replaced by a different amino acid, as indicated by underline in Table 2. In SEQ ID NOs: 12-14, 3 positions selected from positions 73, 74, 82, 138, 141 of SEQ ID NO: 1 are replaced by a different amino acid, as indicated by underline in Table 2.

**Table 2. Binding analysis and temperature stability of Her2 binding proteins**

| | | amino acid | amino acid | amino acid | amino acid | amino acid | SPR | DSF |
|---|---|---|---|---|---|---|---|---|
| SEQ ID NO: | Affilin- | 73 | 74 | 82 | 138 | 141 | K_{D}_hHER2 | Thermal transition |
| 1 | 206478 | L | R | K | Q | S | 0.45 nM | 69.34 °C |
| 2 | 206479 | L | R | K | Q | A | 0.25 nM | 69.40 °C |
| 3 | 206480 | L | R | K | S | S | 0.38 nM | 68.62 °C |
| 4 | 206494 | W | R | K | Q | S | 0.79 nM | 69.60 °C |
| 5 | 206486 | L | Y | K | Q | S | 1.86 nM | 64.40 °C |
| 6 | 206482 | L | R | T | Q | S | 0.27 nM | 66.93 °C |
| 7 | 206483 | L | R | T | Q | A | 0.14 nM | 66.80 °C |
| 8 | 206498 | W | R | T | Q | S | 0.51 nM | 66.81 °C |
| 9 | 206481 | L | R | K | S | A | 0.25 nM | 68.35 °C |
| 10 | 206495 | W | R | K | Q | A | 0.58 nM | 69.40 °C |
| 12 | 206485 | L | R | T | S | A | 0.21 nM | 65.54 °C |
| 13 | 206499 | W | R | T | Q | A | 0.33 nM | 66.60 °C |
| 14 | 206497 | W | R | K | S | A | 0.48 nM | 68.50 °C |

### Example 6. Serum stability of Her2 binding proteins (cell binding assay)

Dilution series from 1 µM to 6 pM of Affilin-206478, Affilin-206479, Affilin-206494, Affilin-206486, Affilin-206495, Affilin-206499, Affilin-206497 (SEQ ID NO: 1, 2, 4, 5, 10, 13, 14) were incubated in 100 % mouse serum for 24 h at 37 °C. Her2 expressing SK-BR-3-cells were trypsinyzed, washed with FACS blocking buffer (PBS, 0.1 % SodiumAcite, 3 % FCS) and seeded in 96-well round bottom plates with a density of 0.1 Mio cells/100 µl. Dilution series of Affilin proteins were incubated with SK-BR-3-cells after 24 h and 0 h (control) in the presence of serum. After 45 min the supernatants were removed and 100 µl/well rabbit anti-Strep-Tag antibody (obtained from GenScript; A00626), 1:300 diluted in FACS blocking buffer were added. After removal of the primary antibody goat anti-rabbit IgG Alexa Fluor 488 antibody (obtained from Invitrogen; A11008) was applied in a 1:1000 dilution. Flow cytometry measurement was conducted on the Guava easyCyte 5HT device from Merck-Millipore at excitation wavelength 488 nm and emission wavelength 525/30 nm. Results are shown in **Table 3.**

**Table 3. Analysis of serum stability of Her2 binding proteins by cell binding assay and ELISA**

| | | Cell binding assay | | | ELISA | | |
|---|---|---|---|---|---|---|---|
| SEQ ID NO | Affilin | K_{D} in nM (0 h serum incubation) | K_{D} in nM (24 h serum incubation) | change K_{D} (-fold) | K_{D} in nM (0 h serum incubation) | K_{D} in nM (24 h serum incubation) | change K_{D} (-fold) |
| 1 | 206478 | 1.35 | 2.66 | 1.97 | 0.29 | 0.33 | 1.14 |
| 2 | 206479 | 1.55 | 1.97 | 1.27 | 0.22 | 0.19 | 0.86 |
| 4 | 206494 | 0.99 | 1.80 | 1.82 | 0.23 | 0.24 | 1.04 |
| 5 | 206486 | 1.23 | 2.31 | 1.88 | 0.33 | 0.25 | 0.75 |
| 10 | 206495 | 1.24 | 2.83 | 2.28 | 0.23 | 0.21 | 0.91 |
| 13 | 206499 | 1.36 | 2.83 | 2.08 | 0.18 | 0.18 | 1.01 |
| 14 | 206497 | 1.44 | 2.23 | 1.55 | 0.23 | 0.21 | 0.90 |

### Example 7. Serum stability of Her2 binding proteins (ELISA)

High binding plates (Greiner, 781061) were immobilized with 1.2 µg/ml recombinant human ErbB2/Her2 Fc Chimera (R&DSystems; Cat.-Nr.: 1129-ER-050) over night at 4 °C. Dilution series of 50 nM to 1.5 pM of Affilin-206479, Affilin-206494, Affilin-206486, Affilin-206495, Affilin-206499, Affilin-206497 (SEQ ID NO: 2, 4, 5, 10, 13, 14) and 37 nM to 1.8 pM of Affilin-206478 (SEQ ID NO: 1) were incubated in 100 % mouse serum for 24 h at 37 °C. ELISA-plates were washed 3 times with PBST (PBS+ 0.1 % Tween) and blocked with 3 % BSA/ 0.5 % Tween/ PBS 2 h at RT. Dilution series after 0 h or 24 h incubation in the presence of serum were incubated on ELISA-plates 1 h at rt. After washing with PBST, wells were incubated with biotinylated anti-ubiquitin-antibody (1:1000) for 1 h at rt. The binding was visualized with Streptavidin-HRP (1:5.000). The Her2 binding proteins show no significant change in K_{D} after 24 h serum incubation **(Table 3 and** **FIGURES 2-8****)** . For example, ELISA analysis confirmed the high stability of Affilin-206479 in serum: the binding of Affilin-206479 (SEQ ID NO 2) to Her2-Fc even after 24 h incubation in mouse serum with a K_{D} of 0.19 nM +/- 0.01 nM is comparable to a K_{D} of 0.22+/- 0.01 nM at 0 h incubation in mouse serum **(****FIGURE 2****).** Similar results were obtained for the other Her2 binding proteins **(****FIGURES 3-8****).**

### Example 8. Cell binding and serum stability of Her2-mlgGFc-Fusion (209438)

The serum stability of the fusion protein 209438 (Affilin-206479 fused to IgG-Fc) was analyzed in mouse serum by ELISA (see Example 7). The KD at 0 h serum incubation was 0.125 nM, the KD at 24 h in serum incubation was 0.167 nM **(****FIGURE 9A****);** the stability of the fusion protein is comparable to Affilin-206479 without fusion to IgG-Fc.

The stability of fusion protein 209438 coupled to DOTA-Fe in mouse serum was analyzed on SK-BR-3-cells. Dilution series of Dota-Fe-coupled fusion protein 209438 from 1 µM to 0.07 nM were incubated with 100 % mouse serum for 24 h at 37 °C. Trypsinized SK-BR-3-cells were washed, blocked with blocking buffer (see Example above) and incubated with the dilution series of fusion protein after 24 h serum incubation and 0 h serum incubation as control. The binding of fusion proteins was analyzed by flow cytometry with StrepTactin-DY488 (IBA; Cat. -Nr.: 2-1562-050).

The KD of control was 4.8 nM, after 24 h serum incubation 6.0 nM **(****FIGURE 9****).**

Both fusion proteins are stable in serum for 24 h. The serum stability of 209438-Dota-Fe in mouse serum for 24 h was also confirmed by ELISA (data not shown).

### Example 9. In vivo study with Affilin-206479 (SEQ ID NO: 2) fused to mlgG-Fc Preparation and analyis of fusion protein for in vivo study

The fusion protein was purified by a two step purification. After using affinity chromatography with HiTrap Protein A HP columns (buffer: 20 mM NaH2PO4 pH 7.0, step elution with 100 mM citric acid pH 4.0) a SEC with a Superdex 200 pg 26/600 column has been performed (buffer: 20 mM citric acid, 150 mM NaCl, 1 mM EDTA).

The purified protein was incubated with 150 µM TCEP as reduction agent one hour at room temperature and afterwards desalted in coupling buffer (50 mM HEPES, 150 mM NaCl, 5 mM EDTA pH 7.0) using HiTrap Desalting columns (GE Healthcare/Cytiva). After the labeling with Maleimide-DOTA (CheMatec) with 20-fold molar excess for three hours at room temperature the sample was applied to an anion exchange chromatography column (Resource Q, GE Healthcare/Cytiva). Therefore 20 mM Bis-Tris pH 7.0 was used as loading buffer. The gradient elution was carried out over 30 CV from 0 % to 30 % Buffer B (20 mM Bis-Tris, 1 M NaCl pH 7.0). The final polishing step was performed using a Superdex 200 pg 26/600 column (GE Healthcare/Cytiva, buffer: 100 mM sodium acetate pH 5.8).

All chromatography processing steps were carried out on ÄKTA avant systems (GE Healthcare/Cytiva) that were previously incubated with 1 M NaOH for reducing endotoxin burden and 100 mM EDTA for reducing metal ion contamination. All steps salts and substances with low endotoxin levels were used when possible.

Homogenous species of the DOTA-coupled and purified protein were obtained; no aggregation was detected. Following SDS-PAGE analysis positive fractions were pooled and their protein concentrations were measured. Further analysis included SE-HPLC and RP-HPLC. Protein concentrations were determined by absorbance measurement at 280 nm using the molar absorbent coefficient. Reversed phase chromatography (RP-HPLC) has been performed using a Dionex HPLC system and a PLRP-S (5 µm, 300 Å) column (Agilent). Analytic size exclusion chromatography (SE-HPLC) has been performed using a Dionex HPLC system and a Superdex200 increase 5/150 GL (GE Healthcare).

Thermal transition point was determined by differential scanning fluorimetry using a LightCycler 480 (Roche). Endotoxin level was measured using an Endosafe nexgen-PTS (Charles River) according to manufacturer manual.

Binding analysis was performed using a Biacore 3000 SPR (GE Healthcare) using a CM5 sensor chip and a SPR-32 (Bruker) using a High Capacity Amine sensor chip respectively. Her2-His and recombinant Protein A were immobilized on the surface of the sensor chips and were equilibrated with SPR runny buffer (PBST 0.05 %). Upon binding, protein analyte was accumulated on the surface increasing the refractive index. This change in the refractive index was measured in real time and plotted as response or resonance units versus time. The analytes were applied to the chip in serial dilutions with a flow rate of 30 µl/min. The association was performed for 120 seconds and the dissociation for 120-180 seconds. After each run, the chip surface was regenerated with 30 µl regeneration buffer (10 mM Glycine pH 2.0) and equilibrated with running buffer. Results are shown in **Table 4.**

**TABLE 4.**

| Construct | SE-HPLC | RP-HPLC | DSF | Endotoxin level | SPR vs. Her2-His | SPR vs. rProtein A |
|---|---|---|---|---|---|---|
| 206479 fused to mIgG₂-Fc conjugated to DOTA | 100 % | 95.2 % | 69.5 °C | 0.01 EU/mg | 0.74 nM | 8.5 nM |

### Radiolabeling of DOTA-Compounds with Lu-177

DOTA-compounds were labelled with ¹⁷⁷LuCl₃ at a specific activity of approximately 1 MBq/nmol while showing a radiochemical purity (RCP) ≥ 95 %. The labeling procedure was as follows: about 20 nmol of DOTA-compound were mixed with around 20 MBq of ¹⁷⁷LuCl₃ solution. The reaction mixture was incubated for 15 min at 50 °C after which the radiolabeled conjugate was challenged with 1 mM DTPA final concentration. The chelation efficiency was determined by iTLC eluting in 0.1 M pH 5 citrate buffer. A part of each radiolabelled solution (about 16-18 MBq) was then added to corresponded unlabelled DOTA protein to obtain a specific activity at about 0.50 MBq/nmol and a concentration about 20 nmol/mL as requested for further steps

### Half life extension of the HER2 binding protein by fusion to mlgG-Fc (In vivo study-pharmacokinetic and tissue distribution)

Anesthetized mice with SK-OV-3 xenograft tumors of 150-300 mm³ were injected with 5 ml/kg of a solution containing 100 nmol/kg test article with an activity of 50 MBq/kg. Tissue distribution was performed at 30 min, 6 h, 24 h, 72 h and 168 h. Animals were euthanized by IP overdose of a mixture of ketamine and xylazine followed by exsanguination via intracardiac puncture. Tumor, kidneys and liver were collected into preweighed tubes and counted in a Gamma counter. **FIGURE 10** shows the uptake of Affilin-206479 in percent injected dose per gram tumor tissue over seven days.

## Claims

1. A binding protein for human Her2 comprising an amino acid sequence with at least 97 % identity to SEQ ID NO: 1, wherein the Her2 binding protein has a binding affinity for Her2 of less than 0.5 nM after 24 h incubation in serum as determined by ELISA, and is stable at temperatures of at least 64°C.

2. The binding protein for human Her2 according to claim 1, comprising an amino acid sequence selected from any of SEQ ID NOs: 1-14.

3. A fusion protein comprising the binding protein for human Her2 according to any of the preceding claims.

4. A fusion protein according to claim 3, wherein the fusion protein comprises additionally at least one molecule modulating pharmacokinetics selected optionally from a serum albumin, an albumin-binding protein, an immunoglobulin binding protein, or an immunoglobulin or immunoglobulin fragment, a polysaccharide, an unstructured amino acid sequence comprising amino acids alanine, glycine, serine, proline, or a polyethylene glycol.

5. A fusion protein according to claim 3 or 4, wherein the fusion protein comprises additionally at least one least one diagnostically active moiety, optionally selected from a radionuclide, fluorescent protein, photosensitizer, dye, or enzyme, or any combination of the above,

6. A fusion protein according to claim 3 or 4, wherein the fusion protein comprises additionally at least one least one therapeutically active moiety, optionally selected from a monoclonal antibody or a fragment thereof, a binding protein, a receptor or receptor domain, a radionuclide, a cytotoxic compound, a cytokine, a chemokine, an enzyme, or derivatives thereof, or any combination of the above.

7. The Her2 binding protein according to claim 1 or 2, or the fusion protein according to any one of claims 3-5, for use in diagnostics or treatment of cancer.

8. A nucleic acid molecule encoding the binding protein for human Her2 as defined in any one of claims 1 to 7.

9. A vector comprising the nucleic acid molecule of claim 8.

10. A host cell or a non-human host comprising the binding protein for human Her2 as defined in claim 1 or 2, a nucleic acid as defined in claim 8, and/or a vector of claim 9.

11. A composition comprising the binding protein for human Her2 as defined in claim 1 or 2; the nucleic acid molecule as defined in claim 8; the vector as defined in claim 9; and/ or the host cell as defined claim 10.

12. A method for the production of the binding protein for human Her2 of claim 1 or 2, comprising culturing of the host cell of claim 10 under suitable conditions in order to obtain said Her2 binding protein and optionally isolating said Her2 binding protein.

## Patentansprüche

1. Bindeprotein für menschliches Her2, umfassend eine Aminosäuresequenz, die zu wenigstens 97 % mit SEQ ID NO: 1 identisch ist, wobei das Her2-Bindeprotein nach 24 h Inkubation in Serum eine Bindungsaffinität für Her2 von kleiner 0,5 nM aufweist, wie mit ELISA bestimmt, und bei Temperaturen von wengistens 64 °C stabil ist.

2. Bindeprotein für menschliches Her2 nach Anspruch 1, umfassend eine aus einer von SEQ ID NO: 1-14 ausgewählte Aminosäuresequenz.

3. Fusionsprotein, umfassend das Bindeprotein für menschliches Her2 nach einem der vorhergehenden Ansprüche.

4. Fusionsprotein nach Anspruch 3, wobei das Fusionsprotein zusätzlich wenigstens ein die Pharmakokinetik modulierendes Molekül umfasst, das gegebenenfalls aus einem Serumalbumin, einem Albumin bindenden Protein, einem Immunglobulin-Bindeprotein oder einem Immunglobulin oder Immunglobulinfragment, einem Polysaccharid, einer Aminosäuren Alanin, Glycin, Serin, Prolin umfassenden unstrukturierten Aminosäuresequenz oder einem Polyethylenglykol ausgewählt ist.

5. Fusionsprotein nach Anspruch 3 oder 4, wobei das Fusionsprotein zusätzlich wenigstens eine diagnostisch wirkende Gruppierung umfasst, die gegebenenfalls aus einem Radionuklid, Fluoreszenzprotein, Photosensibilisator, Farbstoff oder Enzym oder einer beliebigen Kombination der Obigen ausgewählt ist.

6. Fusionsprotein nach Anspruch 3 oder 4, wobei das Fusionsprotein zusätzlich wenigstens eine therapeutisch wirkende Gruppierung umfasst, die gegebenenfalls aus einem monoklonalen Antikörper oder einem Fragment davon, einem Bindeprotein, einem Rezeptor bzw. einer Rezeptordomäne, einem Radionuklid, einer zytotoxischen Verbindung, einem Cytokin, einem Chemokin, einem Enzyme oder Derivaten davon oder einer beliebigen Kombination der Obigen ausgewählt ist.

7. Her2-Bindeprotein nach Anspruch 1 oder 2 oder Fusionsprotein nach einem der Ansprüche 3-5, zur Verwendung bei der Diagnostik oder Behandlung von Krebs.

8. Nukleinsäuremolekül, codierend das Bindeprotein für menschliches Her2 mit der in einem der Ansprüche 1 bis 7 angegebenen Bedeutung.

9. Vektor, umfassend das Nukleinsäuremolekül gemäß Anspruch 8.

10. Wirtszelle oder nichtmenschlicher Wirt, umfassend das Bindeprotein für menschliches Her2 mit der in Anspruch 1 oder 2 angegebenen Bedeutung, eine Nukleinsäure mit der in Anspruch 8 angegebenen Bedeutung und/oder einen Vektor nach Anspruch 9.

11. Zusammensetzung, umfassend das Bindeprotein für menschliches Her2 mit der in Anspruch 1 oder 2 angegebenen Bedeutung; das Nukleinsäuremolekül mit der in Anspruch 8 angegebenen Bedeutung; den Vektor mit der in Anspruch 9 angegebenen Bedeutung; und/oder die Wirtszelle mit der in Anspruch 10 angegebenen Bedeutung.

12. Verfahren zur Herstellung des Bindeproteins für menschliches Her2 gemäß Anspruch 1 oder 2, umfassend Kultivieren der Wirtszelle gemäß Anspruch 10 unter geeigneten Bedingungen, um das Her2-Bindeprotein zu erhalten, und gegebenenfalls Isolieren des Her2-Bindeproteins.

## Revendications

1. Protéine de liaison pour Her2 humaine comprenant une séquence d'acides aminés comportant au moins 97 % d'identité avec la SEQ ID NO: 1, la protéine de liaison de Her2 ayant une affinité de liaison pour Her2 inférieure à 0,5 nM après 24 h d'incubation dans du sérum comme déterminée par ELISA, et étant stable à des températures d'au moins 64 °C.

2. Protéine de liaison pour Her2 humaine selon la revendication 1, comprenant une séquence d'acides aminés choisie parmi l'une quelconque des SEQ ID NO: 1 à 14.

3. Protéine de fusion comprenant la protéine de liaison pour Her2 humaine selon l'une quelconque des revendications précédentes.

4. Protéine de fusion selon la revendication 3, la protéine de fusion comprenant de plus au moins une molécule modulant la pharmacocinétique choisie éventuellement parmi une albumine sérique, une protéine de liaison à l'albumine, une protéine de liaison à une immunoglobuline, ou une immunoglobuline ou un fragment d'immunoglobuline, un polysaccharide, une séquence d'acides aminés non structurée comprenant les acides aminés alanine, glycine, sérine, proline, ou un polyéthylène glycol.

5. Protéine de fusion selon la revendication 3 ou 4, la protéine de fusion comprenant de plus au moins un groupement actif sur le plan diagnostique, éventuellement choisi parmi un radionucléide, une protéine fluorescente, un agent photosensibilisant, un colorant, ou une enzyme, ou une quelconque combinaison de ce qui précède.

6. Protéine de fusion selon la revendication 3 ou 4, la protéine de fusion comprenant de plus au moins un groupement actif sur le plan thérapeutique, éventuellement choisi parmi un anticorps monoclonal ou un fragment de celui-ci, une protéine de liaison, un récepteur ou un domaine de récepteur, un radionucléide, un composé cytotoxique, une cytokine, une chimiokine, une enzyme, ou des dérivés correspondants, ou une quelconque combinaison de ce qui précède.

7. Protéine de liaison de Her2 selon la revendication 1 ou 2, ou protéine de fusion selon l'une quelconque des revendications 3 à 5, pour une utilisation dans des diagnostics ou le traitement d'un cancer.

8. Molécule d'acide nucléique codant pour la protéine de liaison pour Her2 humaine telle que définie dans l'une quelconque des revendications 1 à 7.

9. Vecteur comprenant la molécule d'acide nucléique selon la revendication 8.

10. Cellule hôte ou hôte non humain comprenant la protéine de liaison pour Her2 humaine telle que définie dans la revendication 1 ou 2, un acide nucléique tel que défini dans la revendication 8, et/ou un vecteur selon la revendication 9.

11. Composition comprenant la protéine de liaison pour Her2 humaine telle que définie dans la revendication 1 ou 2 ; la molécule d'acide nucléique telle que définie dans la revendication 8 ; le vecteur tel que défini dans la revendication 9 ; et/ou la cellule hôte telle que définie dans la revendication 10.

12. Procédé pour la production de la protéine de liaison pour Her2 humaine selon la revendication 1 ou 2, comprenant la culture de la cellule hôte selon la revendication 10 dans des conditions appropriées afin d'obtenir ladite protéine de liaison de Her2 et éventuellement l'isolement de ladite protéine de liaison de Her2.
